# EUROPEAN PATENT APPLICATION

(11) **EP 4 104 711 A1**
(43) Date of publication of application: **21.12.2022**
(21) Application number: 21179989.5
(22) Date of filing: 17.06.2021
(51) Int. Cl.: A46B 15/00, A61C 17/22, A46B 13/02, A61B 5/145

(54) **POWER TOOTHBRUSH**

(71) Applicant: Koninklijke Philips N.V., 5656 AG Eindhoven (NL)
(72) Inventor: LACROIX, Joyca Petra Wilma, 5656 AE Eindhoven (NL); RMAILE, Amir Hussein, 5656 AE Eindhoven (NL); TASSIGNON, Tom, 5656 AE Eindhoven (NL)
(74) Representative: Philips Intellectual Property & Standards

(57) **Abstract**

A power toothbrush has a toothbrush head and a handle for driving the toothbrush head with a vibratory motion using drive parameters. The apply drive parameters are adapted based on a sensed pH level. In this way, the brushing can be adapted to the acidity levels presents, and thereby reduce the risk of damage to the teeth caused by excessively hard brushing in an acidic environment.

## Description

### FIELD OF THE INVENTION

This invention relates generally to power toothbrushes.

### BACKGROUND OF THE INVENTION

The development of dental caries and tooth erosion is for a large part influenced by the foods a person consumes, because they induce changes in salivary pH levels. When food is digested in the mouth, different types of acid are released which bring down the pH of saliva. Different types of food and drink have a different impact on salivary pH, e.g. soft drinks (pH 3), white wine (pH 4), cheese (pH 5) etc.

Similar to consuming food and drink, taking certain types of medication or vitamin supplements can (temporarily) lower oral pH levels. For example, aspirin is a commonly used medicine for primary and secondary prevention of cardiovascular diseases, which is an acidic medicine, hence associated with lower oral pH levels. Also, asthma medications for children increase the likelihood of caries incidence. In addition, many medications reduce the flow of saliva and cause a condition called dry mouth syndrome. A dry mouth significantly increases the risk of tooth decay, because saliva neutralizes mouth acids that cause tooth decay and contains substances crucial to the repair of tooth enamel that has been damaged by acids. With a dry mouth with less saliva, this process is slower and less effective.

Many people taking these medications are not aware of these effects of medications on their pH levels and the risk for tooth decay.

The normal pH of saliva is in the range 6.7 to 7.4. When the salivary pH level drops, for example below 5.5, the acids start to demineralize the enamel and thereby soften and break down the enamel tissue. The dentin also suffers from lower pH levels. Demineralization and consequential damage sets in at somewhat higher pH levels for enamel than for dentin primarily due to the higher mineral content in enamel. Three phases of attack have been identified, based on the pH of the acid. Acids with pH 1 can cause surface etching when exposed to teeth even for very short time periods. Nanoscale surface softening occurs with short exposure at a pH in the range 2 to 4, but this does not extend to the macroscale. The third and most common form of acid attack is through weak acid (pH in the range 4.5 to 6.9) subsurface dissolution. This, along with bacteria, can lead to the formation of carious lesions and as a result has been well studied.

As a result of the reduced enamel and dentin hardness, brushing teeth when acidity levels are increased can increase the damaging effect on enamel, and the level of damage will depend on the tooth brushing frequency, force of brushing, type of toothbrush. For example, brushing with the higher frequency movement of sonic toothbrushes is found to correlate with an increased abrasion of dentin.

Usually people are not aware of how food intake and medication intake will affect the pH levels in their mouth. They are also unaware of their current pH levels, and how they may cause damage to their teeth when brushing.

It has been proposed to incorporate a pH sensor into a toothbrush, for example in CN 110269388, so that a user can be made more aware of information relating to their oral health. However, there is no guidance for the user as to how to respond to this information.

### SUMMARY OF THE INVENTION

The invention is defined by the claims.

According to examples in accordance with an aspect of the invention, there is provided a power toothbrush comprising:
a toothbrush head;
a handle to which the toothbrush head is coupled, comprising a drive arrangement for driving the toothbrush head with a vibratory motion using drive parameters;
a pH sensor for sensing an oral pH level; and
a controller, wherein the controller is adapted to apply drive parameters which depend on the sensed pH level.

The invention is based on the recognition that when acidity levels are higher in the mouth, tooth health can benefit from a different brushing profile, to prevent damage to teeth due to brushing.

The power toothbrush thus addresses the problem of serious damage to enamel and dentin when salivary pH levels are below a critical level and the enamel and dentin are demineralized and therefore softer. This is typically the case during a period after food or drink have been consumed, or specific types of medications or vitamin supplements have been taken.

The power toothbrush is able to perform real-time in-mouth pH monitoring, for example via the toothbrush head, and then implements automatic adaptation of the brushing profile when acidity levels are such that the usual movements of the brush are likely to cause damage to enamel and/or dentin. The drive parameters that are adapted may be the motion drive parameters such as vibratory frequency or force and/or feedback parameters such as a force threshold.

The power toothbrush may additionally provide feedback and coaching to the user (e.g. via a remote user interface device) so that the user is educated and coached to make adaptations to his/her brushing routine.

The drive parameters may be set by the controller, or the determination of the suitable drive parameters may be made externally to the toothbrush itself, for example using a processor of a smartphone or other external user device with which the controller of the power toothbrush communicates. In such a system, the processor of the external device and the controller of the toothbrush may together be considered to be a controller for implementing the control of the invention.

The controller within the toothbrush may instead be adapted to determine the drive parameters. In this case, the processing of sensor data is implemented by the toothbrush itself.

The controller (internal or external) may be adapted to compare the sensed pH level with a pH threshold level, and if the sensed pH level is below the pH threshold, to set the drive parameters to a more gentle brushing mode.

Thus, the invention in this aspect is based on the recognition that when acidity levels are higher in the mouth (i.e. a lower pH level) tooth health can benefit from a more gentle type of brushing, to prevent damage to teeth due to brushing. The pH threshold level is for example in the range 5 to 6, such as 5.5. The controller may even switch off the brush if the pH level is such that a risk of tooth damage is too high.

The controller may be adapted to set the drive parameters to a more gentle brushing mode by lowering a frequency or amplitude of the vibrations.

In this way, the vibration frequency or movement amplitude of the toothbrush head are adapted to levels that will lead to less abrasion of teeth, when there are increased acidity levels in the mouth. This prevents the additional damaging effect on enamel and dentin due to brushing in these circumstances.

The controller may instead be adapted to set the drive parameters to a more gentle brushing mode by ending the brushing and providing a recommendation to the user to switch to manual brushing or to change a toothbrush head. A manual brushing is more gentle than use of a power toothbrush, so it may be appropriate to switch to manual brushing when pH levels are low. Similarly, a different toothbrush head may be suitable.

The power toothbrush may further comprise a force or pressure sensor for detecting a brushing force or pressure applied by the toothbrush head. The use of a pressure-sensing mechanism in toothbrushes, both power and manual, is generally well known, and has been implemented in a variety of specific embodiments.

The drive parameters for example include a force or pressure threshold, and the controller is adapted to compare the sensed brushing force or pressure with the force or pressure threshold, and if the force or pressure threshold is reached, to:
providing a warning; or
reduce the applied force or pressure.

The power toothbrush thus performs real-time brushing force monitoring, and a threshold is set to ensure that (or advise that) the user does not exceed a desired brushing force.

The controller may be adapted to set the force or pressure threshold in dependence on the sensed pH level. The sensed pH level is in this way used to adapt the pressure threshold level of the toothbrush. The pressure threshold level is thereby personalized to the pH level in the mouth of the user ensuring that the user is not exerting too much pressure on the teeth based on the pH level detected in real time.

The controller may further comprise an input for receiving information about food and drink intake, and is adapted to provide a recommendation of when to perform brushing in dependence on the food and drink intake.

The food and drink intake may for example be input to a separate device to the toothbrush, and this separate device (e.g. a smartphone with a suitable app) then provides suitable information to the controller of the toothbrush itself for the recommendation to be made.

By tracking timing of intake of different foods, the toothbrush can measure the impact on pH levels and how quickly they are restored. This can serve as input for personalized coaching about timing of brushing to prevent unnecessary damaging of the enamel. For example, the toothbrush can then provide (via a user interface) education and guidance making the user aware of the impact of the food and drink intake on pH levels in the mouth and provide brushing advice, such as brushing with lower brushing force, postponing brushing times or other oral hygiene measures.

The controller may further comprise an input for receiving information about medication or supplement intake, and is adapted to provide brushing recommendations in dependence on the medication or supplement intake.

The information for example comprises medication or supplement intake times and doses, and the pH level can be sensed at different moments (when brushing) to derive the impact of the medication on pH levels. The toothbrush can again provide (via a user interface) education and guidance making the user aware of the impact of the medication on pH levels in the mouth and provide brushing advice, such as brushing with lower brushing force, postponing brushing times or other oral hygiene measures.

The power toothbrush may comprise a wireless communications system for providing the sensed pH level to an external device and receiving the drive parameters from the external device.

The external device is for example a mobile phone on which an app is loaded to perform the processing of sensor data and function as the user interface device for the user.

The invention also provides a method of setting drive parameters of a power toothbrush, wherein the power toothbrush comprises a toothbrush head and handle to which the toothbrush head is coupled, comprising a drive arrangement for driving the toothbrush head with a vibratory motion using drive parameters, the method comprising:
receiving a sensed oral pH level; and
setting the drive parameters in dependence on the sensed pH level.

This is the method implemented by the power toothbrush defined above.

The method may comprise comparing the sensed pH level with a pH threshold level, and if the sensed pH level is below the pH threshold, to set the drive parameters to a more gentle brushing mode, by lowering a frequency or amplitude of vibrations of the brushing head.

The method may further comprise receiving a detected brushing force or pressure, comparing the sensed brushing force or pressure with a force or pressure threshold level, and if the force or pressure threshold is reached, to:
providing a warning; or
reduce the applied force or pressure,
wherein the method comprises setting the force or pressure threshold in dependence on the sensed pH level.

The invention also provides a computer program comprising computer program code which is adapted, when said program is run on a controller of a power toothbrush, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a toothbrush system; and
Figure 2 shows a method of setting drive parameters of a power toothbrush.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a power toothbrush having a toothbrush head and a handle for driving the toothbrush head with a vibratory motion using drive parameters. The apply drive parameters are adapted based on a sensed pH level. In this way, the brushing can be adapted to the acidity levels presents, and thereby reduce the risk of damage to the teeth caused by excessively hard brushing in an acidic environment.

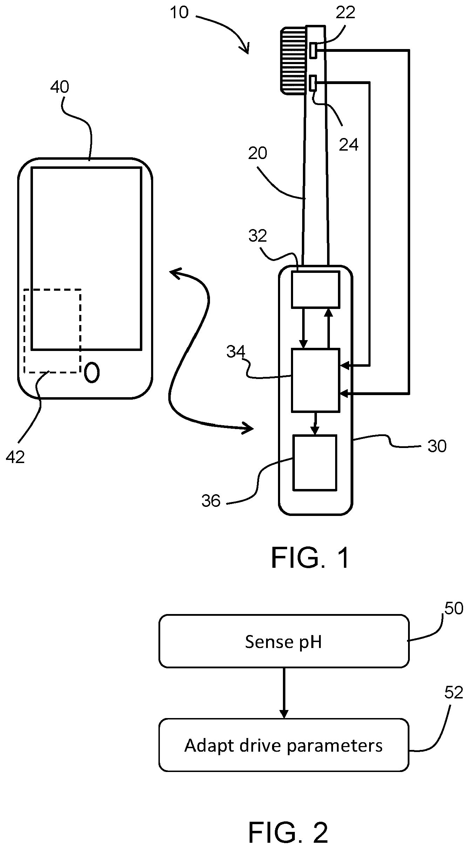

Figure 1 shows a toothbrush system comprising a power toothbrush 10 and a remote device 40. The power toothbrush comprises a toothbrush head 20 and a handle 30 to which the toothbrush head is coupled. The handle 30 has a drive arrangement 32 for driving the toothbrush head with a vibratory motion using drive parameters. The drive arrangement is controlled by a controller 34.

The toothbrush also comprises a pH sensor 22 for sensing an oral pH level. The pH sensor is positioned to detect the pH in the oral cavity resulting for example from the types of food or medication that have been consumed by the user. The location is thus for example away from the location of the bristles so that an influence of the toothpaste on a pH measurement is minimized. The pH sensor is for example exposed to the back of the toothbrush head 20.

The controller 34 applies drive parameters which depend on the sensed pH level. In particular, when acidity levels are higher in the mouth, tooth health can benefit from a different brushing profile, to prevent damage to teeth due to brushing. The controller for example applies a threshold to the sensed pH level representing a critical level at which enamel and dentin are demineralized and therefore softer. If the sensed pH level is below the pH threshold (corresponding to high acidity levels), the drive parameters are then set to a more gentle brushing mode. Automatic adaptation of the brushing profile may then be implemented when acidity levels are such that the usual movements of the brush are likely to cause damage to enamel and/or dentin. The pH threshold level is for example in the range 5 to 6, such as 5.5.

A more gentle brushing mode may for example be implemented by lowering a frequency or amplitude of the vibrations generated by the drive arrangement. In this way, the vibration frequency or movement amplitude of the toothbrush head are adapted to levels that will lead to less abrasion of teeth, when there are increased acidity levels in the mouth. This prevents the additional damaging effect on enamel and dentin due to brushing in these circumstances.

The controller may even switch off the brush if the pH level is such that a risk of tooth damage is too high. The toothbrush may then provide a recommendation to the user to switch to manual brushing or to change a toothbrush head. A manual brushing is more gentle than use of a power toothbrush, so it may be appropriate to switch to manual brushing when pH levels are low. Similarly, a different toothbrush head may be suitable.

Figure 1 also shows a force or pressure sensor 24 for detecting a brushing force or pressure applied by the toothbrush head. The use of pressure sensing in toothbrushes is well known. A pressure sensor for example comprises a simple spring, a moment arm and a switch. As pressure increases, typically due to the action of the user pressing the toothbrush against the teeth, the spring is compressed, which moves the moment arm. Magnetic pressure sensors are also known using a magnet positioned so that it moves in accordance with the brush head assembly motion. A Hall effect sensor is mounted within the magnetic field generated by the magnet. The magnetic field response has a phase shift relative to the phase of the drive signal so that a determined phase shift of the Hall sensor output can be determined and is indicative of the load on the brush element during brushing.

Thus, there are various known ways to monitor the pressure applied to the teeth during brushing, and thereby detect the brushing characteristics, in particular the brushing force applied by the user of the power toothbrush.

The toothbrush may use the pressure sensing functionality in an entirely conventional manner. For example, the drive parameters set by the drive arrangement 32 may include a force or pressure threshold. The controller 34 may then compare the sensed brushing force or pressure with the force or pressure threshold. If the force or pressure threshold is reached a warning may be generated, i.e. an output signal to a user via a user interface. In addition, the force or pressure applied may be reduced by adapting the drive parameters (preferably down to the threshold if this can be achieved by adapting the drive parameters alone). Thus, the usual movements of the toothbrush are reduced if brushing is determined to be too hard. The power toothbrush thus performs real-time brushing force monitoring, and a threshold is set to ensure that (or advise that) the user does not exceed a desired brushing force. This approach is well known.

The invention enables a refinement to the force or pressure feedback system. The pressure sensing functionality may additionally combine with the pH sensing, so that the force or pressure threshold is set in dependence on the sensed pH level. The sensed pH level is in this way used to adapt the pressure threshold level of the toothbrush. The pressure threshold level is thereby personalized to the pH level in the mouth of the user ensuring that the user is not exerting too much pressure on the teeth based on the pH level detected in real time. In this way, over-pressure threshold levels in the toothbrush are adapted in real time, thereby to reduce the likelihood of causing damage to enamel and/or dentin. This approach may be instead of, or as well as changing the movement drive parameters.

Figure 1 shows that the toothbrush may form part of an overall system which additionally includes an external user device 40 such as a mobile phone (smart phone). The toothbrush has RF communications circuity 36 for communicating wirelessly with the mobile phone 40.

The overall system may thus be considered to have a controller which is the combination of the local controller 34 in the toothbrush and the processor of the mobile phone.

The mobile phone functions as a user interface device, based on an app loaded on the mobile phone and run by a processor 42 of the mobile phone 40. In addition, the processing of the sensed pH level, to determine how to adapt the drive parameters and/or generate a warning, may be performed either by the processor 34 of the toothbrush or by the processor 42 of the mobile phone 40 or even by another remote processor with which the toothbrush and/or mobile phone communicate. The toothbrush will always have a local controller for example for applying the drive parameters to the drive arrangement.

The user interface function enabled by the mobile phone may be used to enable the user to input information about food and drink intake. The system may then provide a recommendation of when to perform brushing in dependence on the food and drink intake.

By tracking timing of intake of different foods, the toothbrush can measure the impact on pH levels and how quickly they are restored. This can serve as input for personalized coaching about timing of brushing to prevent unnecessary damaging of the enamel. For example, the toothbrush can then provide (via a user interface) education and guidance making the user aware of the impact of the food and drink intake on pH levels in the mouth and provide brushing advice, such as brushing with lower brushing force, postponing brushing times or other oral hygiene measures.

Similarly, the user interface function may enable the user to provide information about medication or supplement intake. The system may then provide brushing recommendations which also depend on the medication or supplement intake. Based on medication or supplement intake times and doses, the pH level can be sensed at different moments (when brushing) to derive the impact of the medication on pH levels. A machine learning algorithm may be used to derive the information relating to the impact of the medication on pH levels.

The toothbrush can again provide education relating to the impact of the medication on pH levels in the mouth and provide brushing advice as outlined above. For a medication that gives a dry mouth, advice may be given to drink more water during the day so that saliva is better able to restore pH levels in the mouth.

As explained above, there are various possible implementations of the system. In one preferred implementation of the system, the brush head has built-in pH monitoring and the toothbrush performs real-time pH monitoring. The sensed raw data is sent to the user's smart phone 40, which runs an algorithm (app) that compares the measured pH levels to a pre-set threshold (e.g. 5.5). If the pH level is below the threshold, the algorithm in the mobile phone instructs the brush to proceed to a more gentle mode of brushing with a lower number of vibrations per second.

Optionally, feedback is also given to the user advising to lower the brushing force. This may use the mobile phone 40 but equally a feedback message may be conveyed using characteristic vibrations or light signals generated by the brush. The brush can also switch off automatically and the user is then instructed to postpone the brushing to a later moment (when pH levels are restored) or continue manually with a low brushing force.

Figure 2 shows a method of setting drive parameters of a power toothbrush, wherein the power toothbrush comprises a toothbrush head and handle, to which the toothbrush head is coupled, comprising a drive arrangement for driving the toothbrush head with a vibratory motion using drive parameters. The method comprises receiving a sensed oral pH level in step 50 and setting the drive parameters in dependence on the sensed pH level in step 52.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A power toothbrush (10) comprising:
a toothbrush head (20);
a handle (30) to which the toothbrush head is coupled, comprising a drive arrangement (32) for driving the toothbrush head with a vibratory motion using drive parameters;
a pH sensor (22) for sensing an oral pH level; and
a controller (34), wherein the controller is adapted to apply drive parameters which depend on the sensed pH level.

2. The power toothbrush of claim 1, wherein the controller (34) is also adapted to determine the drive parameters.

3. The power toothbrush of claim 2, wherein the controller (34) is adapted to:
compare the sensed pH level with a pH threshold level, and if the sensed pH level is below the pH threshold, to set the drive parameters to a more gentle brushing mode.

4. The power toothbrush of claim 3, wherein the controller (34) is adapted to set the drive parameters to a more gentle brushing mode by lowering a frequency or amplitude of the vibrations.

5. The power toothbrush of claim 3, wherein the controller (34) is adapted to set the drive parameters to a more gentle brushing mode by ending the brushing and providing a recommendation to the user to switch to manual brushing or to change a toothbrush head.

6. The power toothbrush of any one of claims 1 to 5, further comprising a force or pressure sensor (24) for detecting a brushing force or pressure applied by the toothbrush head.

7. The power toothbrush of claim 6, wherein the drive parameters include a force or pressure threshold, wherein the controller (34) is adapted to compare the sensed brushing force or pressure with the force or pressure threshold, and if the force or pressure threshold is reached, to:
providing a warning; or
reduce the applied force or pressure.

8. The power toothbrush of claim 7, wherein the controller (34) is adapted to set the force or pressure threshold in dependence on the sensed pH level.

9. The power toothbrush of any one of claims 1 to 8, wherein the controller (34) further comprises an input for receiving information about food and drink intake, and is adapted to provide a recommendation of when to perform brushing in dependence on the food and drink intake.

10. The power toothbrush of any one of claims 1 to 9, wherein the controller (34) further comprises an input for receiving information about medication or supplement intake, and is adapted to provide brushing recommendations in dependence on the medication or supplement intake.

11. The power toothbrush of any one of claims 1 or 6 to 10, comprising a wireless communications system (36) for providing the sensed pH level to an external device (40) and receiving the drive parameters from the external device (40).

12. A method of setting drive parameters of a power toothbrush, wherein the power toothbrush comprises a toothbrush head and handle to which the toothbrush head is coupled, comprising a drive arrangement for driving the toothbrush head with a vibratory motion using drive parameters, the method comprising:
(50) receiving a sensed oral pH level; and
(52) setting the drive parameters in dependence on the sensed pH level.

13. The method of claim 12, comprising comparing the sensed pH level with a pH threshold level, and if the sensed pH level is below the pH threshold, to set the drive parameters to a more gentle brushing mode, by lowering a frequency or amplitude of vibrations of the brushing head.

14. The method of claims 12 or 13, further comprising receiving a detected brushing force or pressure, comparing the sensed brushing force or pressure with a force or pressure threshold level, and if the force or pressure threshold is reached, to:
providing a warning; or
reduce the applied force or pressure,
wherein the method comprises setting the force or pressure threshold in dependence on the sensed pH level.

15. A computer program comprising computer program code which is adapted, when said program is run on a controller of a power toothbrush, to implement the method of any one of claims 12 to 14.
